# EUROPÄISCHE PATENTANMELDUNG

(11) **EP 0 606 049 A1**
(43) Veröffentlichungstag der Anmeldung: **13.07.1994**
(21) Anmeldenummer: 93810905.5
(22) Anmeldetag: 24.12.1993
(51) Int. Cl.: C07D 295/18, A61K 31/495

(54) **Aminoalkylphenyl-Verbindungen**

(30) Priorität: 06.01.1993 CH 25/93
(71) Anmelder: CIBA-GEIGY AG, CH-4002 Basel (CH)
(72) Erfinder: Ferrini, Pier Giorgio, Dr., CH-4102 Binningen (CH)

(57) **Zusammenfassung**

Aminoalkylphenyl-Derivate der Formel I, 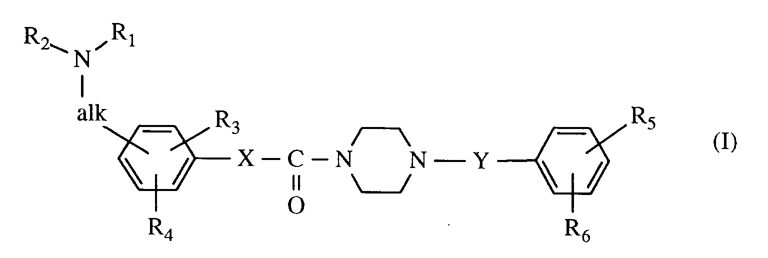
worin alk, R₁ - R₆, X und Y wie in der Beschreibung definiert sind, sowie Salze davon, weisen die Biosynthese von Interleukin-1 (IL-1) hemmende sowie analgetische Eigenschaften auf und können daher als Arzneimittelwirkstoffe verwendet werden. Sie werden in an sich bekannter Weise hergestellt.

## Beschreibung

Die Erfindung betrifft neue Aminoalkylphenyl-Derivate der Formel I, 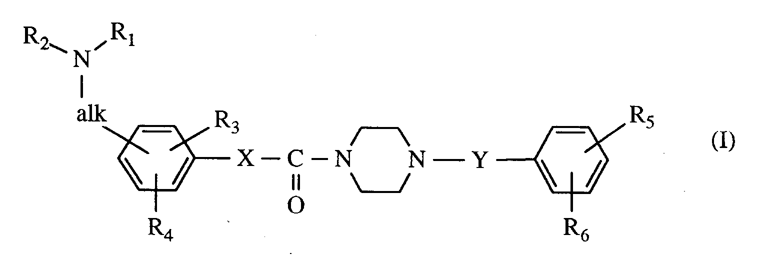
worin alk Niederalkylen bedeutet, R₁ für Wasserstoff, Niederalkyl, Niederalkoxy-niederalkyl, Niederalkoxy-niederalkoxy-niederalkyl, Aryloxy-niederalkyl, Arylnieder-alkoxy-niederalkyl, N-Niederalkylamino-niederalkyl oder N,N-Diniederalkylamino-niederalkyl steht, R₂ Niederalkenoyl, (Carboxy oder funktionell modifiziertes Carboxy)-niederalkenoyl, elektronegativ substituiertes Niederalkanoyl, Carbamoyl, N-Niederalkylcarbamoyl, N,N-Diniederalkylcarbamoyl, Carboxy-carbonyl, Niederalkoxycarbonyl-carbonyl, (Carbamoyl, N-Niederalkylcarbamoyl, N,N-Diniederalkylcarbamoyl)-carbonyl, Wasserstoff, Niederalkyl, Niederalkanoyl, Carboxy, Niederalkoxycarbonyl oder Arylniederalkoxycarbonyl bedeutet, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, und X und Y unabhängig voneinander eine direkte Bindung, Niederalkylen oder Niederalkenylen bedeuten, und Salze davon, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Elektronegativ substituiertes Niederalkanoyl bedeutet z.B. Niederalkanoyl, das durch Halogen, Amino, Niederalkylamino, (Carboxy oder Niederalkoxycarbonyl)-niederalkylamino, Diniederalkylamino; 4- bis 6-gliedriges Niederalkylenamino, z.B. Piperidino; Morpholino, Thiomorpholino, Piperazino - gegebenenfalls in 4-Stellung Niederalkyl- oder Niederalkanoyl-substituiert -, Hydroxy, Niederalkoxy, Acyloxy, Niederalkylthio, Niederalkylsulfinyl, Niederalkylsulfonyl, Arylthio, Arylsulfinyl, Arylsulfonyl, Carboxy, Niederalkoxycarbonyl, Carbamoyl, N-Niederalkylcarbamoyl, N,N-Diniederalkylcarbamoyl oder Cyano substituiert ist.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkylen ist insbesondere C₁-C₇-Alkylen, z.B. Methylen; Aethylen, etwa 1,2- oder 1,1-Aethylen; Propylen, etwa 1,3-, 1,2- oder 1,1-Propylen; Butylen, Pentylen, Hexylen oder Heptylen; vorzugsweise C₁-C₄-Alkylen und in erster Linie Methylen oder 1,2-Aethylen.

Niederalkyl ist beispielsweise C₁-C₇-Alkyl, vorzugsweise C₁-C₄-Alkyl, wie insbesondere Methyl oder in zweiter Linie Äthyl, n-Propyl, Isopropyl oder n-Butyl, kann aber z.B. auch Isobutyl, sek.-Butyl, tert.-Butyl oder eine Pentyl-, Hexyl- oder Heptylgruppe sein.

### Halogen-niederalkyl ist z.B. Trifluormethyl.

Niederalkenoyl ist z.B. C₃-C₇-Alkenoyl, vorzugsweise C₃-C₅-Alkenoyl, wie Prop-2-enoyl (Acryloyl), 2-Methylprop-2-enoyl (Methacryloyl), But-2-enoyl, But-3-enoyl 3-Methylbut-3-enoyl oder Pent-4-enoyl.

(Carboxy oder funktionell modifiziertes Carboxy)-niederalkenoyl ist z.B. Niederalkenoyl, das durch Carboxy, Niederalkoxycarbonyl, Carbamoyl, N-Niederalkylcarbamoyl, N,N-Diniederalkylcarbamoyl oder Cyano, insbesondere durch Carboxy oder Niederalkoxycarbonyl, substituiert ist.

Halogen ist insbesondere Chlor, Fluor oder Brom, kann aber auch für Iod stehen.

Halogen-niederalkanoyl ist beispielsweise Halogen-C₂-C₇-alkanoyl, wobei Halogen insbesondere Chlor bedeutet, aber in zweiter Linie auch Fluor oder Brom sein kann, vorzugsweise Chlor-C₂-C₄-alkanoyl, wie Chloracetyl, 3-Chlorpropionyl oder 4-Chlorbutyryl.

Diniederalkylamino-niederalkanoyl ist z.B. Di-C₁-C₄-Alkylamino-C₂-C₇-alkanoyl, und vorzugsweise N,N-Dimethylamino-C₂-C₄-alkanoyl, wie Dimethylaminoacetyl.

4- bis 6-Gliedriges Niederalkylenamino-niederalkanoyl ist z.B. Pyrrolidino- und vorzugsweise Piperidino-C₂-C₄-alkanoyl, etwa Piperidinoacetyl.

Niederalkoxy-niederalkyl trägt die Niederalkoxygruppe vorzugsweise in höherer als der α-Stellung und ist beispielsweise entsprechendes C₁-C₄-Alkoxy-C₂-C₄-alkyl, wie 2-Methoxyäthyl, 2-Äthoxyäthyl, 2-Propyloxyäthyl, 2-Isopropyloxyäthyl, 3-Methoxypropyl, 3-Äthoxypropyl, 3-Isopropyloxypropyl oder 4-Methoxybutyl.

Carboxy-carbonyl steht für die Gruppe -C(=O)-COOH.

Aryl ist z.B. Phenyl, das unsubstituiert oder durch einen oder zwei Substituenten aus der Gruppe bestehend aus Niederalkyl, Niederalkoxy, Hydroxy, Halogen und Trifluormethyl substituiert ist, und vor allen Dingen Phenyl.

Acyl ist z.B. Niederalkanoyl.

Salze von Verbindungen der Formel I sind insbesondere pharmazeutisch verwendbare Salze, z.B. Säureadditionssalze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren oder gegebenenfalls ungesättigten oder hydroxylierten aliphatischen Dicarbonsäuren, z.B. Acetate, Oxalate, Malonate, Maleinate, Fumarate, Maleate, Tartrate oder Citronate.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel I und ihre pharmazeutisch verwendbaren Salze weisen wertvolle pharmakologische Eigenschaften auf. Insbesondere zeigen sie ausgeprägte Hemmwirkung auf die Biosynthese von Interleukin-1 (IL-1). IL-1 gehört zur Klasse der proinflammatorischen Proteine und spielt beispielsweise bei der Prostaglandinsynthese, der Synthese neutraler Proteasen durch Fibroplasten, Synovialzellen und Chondrozyten, bei der Aktivierung von Endothelialzellen und bei der Induktion weiterer proinflammatorischer Zytokine, wie des α-Tumornekrosefaktors (TNF) und von Interleukin-6 (IL-6) eine wesentliche Rolle. Ferner regt es die Knochenresorption an, reguliert die Körpertemperatur von Warmblütern und reguliert u.a. die Entwicklung, Aktivierung, Differenzierung und Proliferation von Lymphozyten. Therapeutisch besonders wichtig ist die Hemmwirkung der Verbindungen der Formel I und ihrer pharmazeutisch verwendbaren Salze auf die Biosynthese von IL-1, TNF und IL-6. Diese kann in vitro beispielsweise an durch Lipopolysaccharide stimulierten (LPS-stimulierten) menschlichen Monozyten nach C. Rordorf-Adam et al., Drugs Exptl. Clin. Res. XV, 355-62(1989) im Konzentrationsbereich ab etwa 0,1 µM und in vivo in der Maus anhand der Hemmung der LPS-induzierten Bildung von Serumamyloid P (SAP) bei einer ED₅₀ von etwa 1 bis 15 mg/kg p.o. und an der Ratte anhand der Senkung des durch LPS erzeugten künstlichen Fiebers bei einer ED₅₀ von etwa 0,05 bis 3,5 mg/kg p.o. gezeigt werden.

Aufgrund dieser Eigenschaften sind die Verbindungen der Formel I und ihre pharmazeutisch verwendbaren Salze vorzüglich geeignet zur therapeutischen Behandlung von Erkrankungen, bei denen eine übermässige Produktion von IL-1 eine ursächliche oder verschlimmernde Rolle spielt, wie entzündliche und degenerative Gelenkerkrankungen, beispielsweise rheumatoide Arthritis, Osteoarthrosis, psoriatische oder infektiöse Arthritis, das Reitersyndrom, Gicht und traumatische Arthritis, und andere akute oder chronische Entzündungen, beispielsweise entzündliche Darmerkrankungen, Meningitis, Hauterkrankungen, z.B. Psoriasis oder Pemphigus vulgaris, allergische Hautreaktionen, Atherosklerose und Autoimmunerkrankungen, wie Diabetes (Typ 1) und Thyroiditis.

Als weitere Erkrankungen, bei denen eine übermässige Produktion von IL-1 eine ursächliche oder verschlimmernde Rolle spielt, sind z.B. zu nennen: Knochenmetabolismus-Regulationsstörungen, z.B. Paget-Krankheit, Osteoporose, Periodontitis oder Malignitäten; oder endotoxischer Schock, z.B. verbunden mit Fieber, Hypotension und fulminantem Leberversagen.

Zusätzlich weisen die Verbindungen der Formel I und ihre pharmazeutisch verwendbaren Salze eine ausgeprägte analgetische Wirkung auf, die sich beispielsweise anhand der Hemmung des durch Phenyl-p-benzochinon ausgelösten Writhingsyndroms der Maus beispielsweise in einer an Hendershot und Forsaith, J. Pharmacol. Exp. Therap. 125, 237 (1959) angelehnten Versuchanordnung mit einer ED₅₀ von etwa 1 bis 30 mg/kg p.o. nachweisen lässt.

Die Verbindungen der Formel I und ihre pharmazeutisch verwendbaren Salze können dementsprechend auch als analgetische Arzneimittelwirkstoffe zur Behandlung von Schmerzzuständen unterschiedlicher Genese, insbesondere als periphere Analgetika, verwendet werden.

Bevorzugt betrifft die Erfindung die Verbindungen der Formel I, worin alk Niederalkylen bedeutet, R₁ für Wasserstoff, Niederalkyl, Niederalkoxy-niederalkyl, Niederalkoxy-niederalkoxy-niederalkyl; Phenyloxy-niederalkyl oder Phenylniederalkoxy-niederalkyl, wobei in den beiden letztgenannten Resten die Phenylgruppe jeweils unsubstituiert oder durch Niederalkyl, Trifluormethyl, Niederalkoxy, Halogen und/oder Hydroxy substituiert ist; N-Niederalkylamino-niederalkyl oder N,N-Diniederalkylamino-niederalkyl steht, R₂ Niederalkenoyl, (Carboxy, Niederalkoxycarbonyl, Carbamoyl, N-Niederalkylcarbamoyl, N,N-Diniederalkylcarbamoyl oder Cyano)-niederalkenoyl, Halogen-niederalkanoyl, Amino-niederalkanoyl, N-Niederalkylamino-niederalkanoyl, N-[(Carboxy oder Niederalkoxycarbonyl)-niederalkylamino]-niederalkanoyl, N,N-Diniederalkylamino-niederalkanoyl, C₄-C₆-Niederalkylenamino-niederalkanoyl, Morpholino-niederalkanoyl, Thiomorpholino-niederalkanoyl; Piperazino-niederalkanoyl, wobei der Piperazinorest unsubstituiert oder in 4-Stellung durch Niederalkyl oder Niederalkanoyl substituiert ist; Hydroxy-niederalkanoyl, Niederalkoxy-niederalkanoyl, Niederalkanoyloxy-niederalkanoyl, Niederalkylthio-niederalkanoyl, Niederalkylsulfinyl-niederalkanoyl, Niederalkylsulfonyl-niederalkanoyl; Phenylthio-niederalkanoyl, Phenylsulfinyl-niederalkanoyl oder Phenylsulfonyl-niederalkanoyl, wobei in den drei letztgenannten Resten die Phenylgruppe jeweils unsubstituiert oder durch Niederalkyl, Niederalkoxy, Halogen und/oder Hydroxy substituiert ist; Carboxy-niederalkanoyl, Niederalkoxy-carbonyl-niederalkanoyl, Carbamoyl-niederalkanoyl, N-Niederalkylcarbamoyl-nieder-alkanoyl, N,N-Diniederalkylcarbamoyl-niederalkanoyl, Cyano-niederalkanoyl, Carbamoyl, N-Niederalkylcarbamoyl, N,N-Diniederalkylcarbamoyl, Carboxy-carbonyl, Niederalkoxycarbonyl-carbonyl, (Carbamoyl, N-Niederalkylcarbamoyl, N,N-Diniederalkylcarbamoyl)-carbonyl, Wasserstoff, Niederalkanoyl, Carboxy, Niederalkoxycarbonyl oder Phenylniederalkoxycarbonyl, worin die Phenylgruppe unsubstituiert oder durch Niederalkyl, Niederalkoxy, Halogen und/oder Hydroxy substituiert ist, bedeutet, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, und X und Y unabhängig voneinander eine direkte Bindung, Niederalkylen oder Niederalkenylen bedeuten, und Salze davon.

Besonders bevorzugt betrifft die Erfindung die Verbindungen der Formel I, worin alk Niederalkylen bedeutet, R₁ für Wasserstoff, Niederalkyl, Niederalkoxy-niederalkyl, Niederalkoxy-niederalkoxy-niederalkyl, Phenyloxy-niederalkyl, N-Niederalkylamino-niederalkyl oder N,N-Diniederalkylamino-niederalkyl steht, R₂ Niederalkenoyl, (Carboxy oder Niederalkoxycarbonyl)-niederalkenoyl, Halogen-niederalkanoyl, N-Niederalkylamino-niederalkanoyl, N-[(Carboxy oder Niederalkoxycarbonyl)-niederalkylamino]-niederalkanoyl, N,N-Diniederalkylamino-niederalkanoyl, Piperidino-niederalkanoyl, Hydroxy-niederalkanoyl, Niederalkoxy-niederalkanoyl, Niederalkanoyloxy-niederalkanoyl, Niederalkylthio-niederalkanoyl, Niederalkylsulfinyl-niederalkanoyl, Niederalkylsulfonyl-niederalkanoyl, Phenylthio-niederalkanoyl, Phenylsulfinyl-niederalkanoyl, Phenylsulfonyl-niederalkanoyl, Carboxy-niederalkanoyl, Niederalkoxycarbonyl-niederalkanoyl, Wasserstoff oder Niederalkanoyl bedeutet, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Trifluormethyl, Niederalkoxy, Niederalkylthio, Halogen oder Diniederalkylamino stehen, und X und Y unabhängig voneinander eine direkte Bindung, Niederalkylen oder Niederalkenylen bedeuten, und Salze davon.

Ganz besonders bevorzugt betrifft die Erfindung die Verbindungen der Formel I, worin alk Methylen oder 1,2-Aethylen bedeutet, R₁ für Wasserstoff, Niederalkyl oder Niederalkoxy-niederalkyl steht, R₂ Niederalkenoyl, (Carboxy oder Niederalkoxycarbonyl)-niederalkenoyl oder Halogen-niederalkanoyl bedeutet oder R₂, sofern R₁ für Niederalkoxy-niederalkyl steht, auch Wasserstoff bedeuten kann, R₃ und R₄ unabhängig voneinander Wasserstoff Niederalkyl, Halogen, Niederalkoxy oder Niederalkylthio bedeuten, R₅ für Niederalkylthio, Chlor, Fluor oder Brom steht, R₆ Wasserstoff bedeutet, X für eine direkte Bindung oder 1,2-Aethenylen steht und Y 1,2-Aethylen bedeutet, und pharmazeutisch verwendbare Salze davon.

Die Erfindung betrifft ferner insbesondere die Verbindungen der Formel Ia, 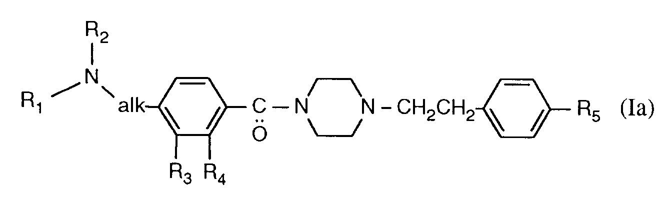
worin alk Niederalkylen bedeutet, R₁ für Wasserstoff, Niederalkyl oder Niederalkoxyniederalkyl steht, R₂ Niederalkenoyl, (Carboxy oder Niederalkoxycarbonyl)-niederalkenoyl oder durch Halogen, Diniederalkylamino, 4- bis 6-gliedriges Niederalkylenamino oder Niederalkylthio substituiertes Niederalkanoyl bedeutet oder R₂, sofern R₁ Niederalkoxyniederalkyl darstellt, Wasserstoff bedeuten kann, R₃ und R₄ unabhängig voneinander Wasserstoff, Niederalkyl, Chlor, Fluor oder Brom bedeuten und R₅ Niederalkylthio, Chlor, Fluor oder Brom darstellt, und Salze davon.

Die Erfindung betrifft vor allem Verbindungen der Formel Ia, worin alk für C₁-C₄-Alkylen steht, R₁ Wasserstoff, C₁-C₄-Alkyl oder C₁-C₄-Alkoxy-C₂-C₄-alkyl bedeutet, R₂ C₃-C₇-Alkenoyl, (Carboxy oder C₁-C₄-Alkoxycarbonyl)-C₃-C₇-alkenoyl, Chlor-C₂-C₄-alkanoyl, C₁-C₄-Alkylthio-, C₁-C₄-Alkylsulfinyl- oder C₁-C₄-Alkylsulfonyl-C₂-C₄-alkanoyl bedeutet oder R₂, sofern R₁ für C₁-C₄-Alkoxy-C₂-C₄-alkyl steht, auch Wasserstoff darstellen kann, R₃ und R₄ unabhängig voneinander Wasserstoff, C₁-C₄-Alkyl, Fluor oder Chlor bedeuten und R₅ für Chlor oder Brom steht, und pharmazeutisch verwendbare Salze davon.

Die Erfindung betrifft insbesondere Verbindungen der Formel Ia, worin alk für Methylen oder 1,2-Aethylen steht, R₁ Wasserstoff, Methyl, Äthyl oder 2-Isopropyloxyäthyl bedeutet, R₂ Prop-2-enoyl, 3-(Carboxy oder C₁-C₄-Alkoxycarbonyl)-prop-2-enoyl, Chloracetyl, 3-Chlorpropionyl, Methylthioacetyl oder Äthylthioacetyl darstellt, R₃ und R₄ unabhängig voneinander Wasserstoff, Methyl, Fluor oder Chlor bedeuten und R₅ für Chlor oder Brom steht, und pharmazeutisch verwendbare Salze davon.

Die Erfindung betrifft in erster Linie Verbindungen der Formel Ia, worin alk für Methylen oder 1,2-Äthylen steht, R₁ Wasserstoff oder 2-Isopropyloxyäthyl bedeutet, R₂ Chloracetyl darstellt, R₃ und R₄ unabhängig voneinander Wasserstoff, Methyl, Fluor oder Chlor bedeuten und R₅ für Chlor oder Brom steht, und pharmazeutisch verwendbare Salze davon.

Die Erfindung betrifft namentlich die in den Beispielen genannten Verbindungen der Formel I und ihre Salze, insbesondere ihre pharmazeutisch verwendbaren Salze.

Das Verfahren zur Herstellung der Verbindungen der Formel I beruht auf an sich bekannten Methoden und ist z.B. dadurch gekennzeichnet, dass man
a) eine Verbindung der Formel II, 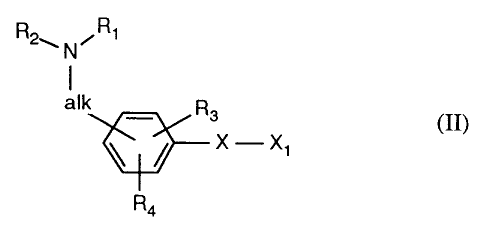 worin X₁ Carboxy oder reaktionsfähiges funktionell abgewandeltes Carboxy bedeutet, oder ein Salz davon, mit einer Verbindung der Formel III, 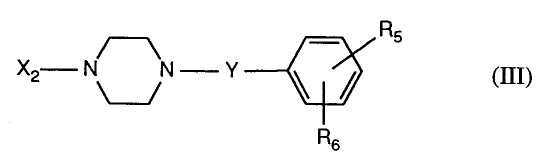 worin X₂ Wasserstoff oder eine Aminoschutzgruppe bedeutet, umsetzt, oder
b) eine Verbindung der Formel IV, 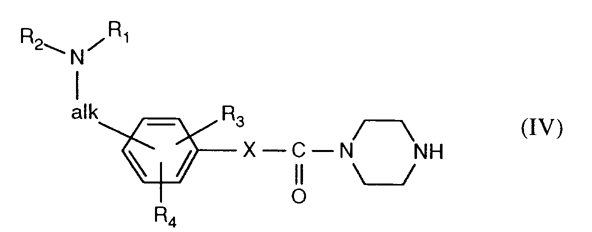 oder ein Salz davon, mit einer Verbindung der Formel V, 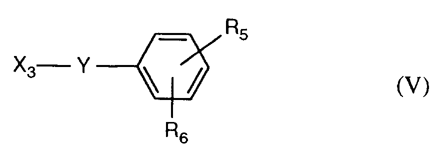 worin X₃ Hydroxy oder reaktionsfähiges verestertes Hydroxy bedeutet, umsetzt, oder
c) eine Verbindung der Formel VI, 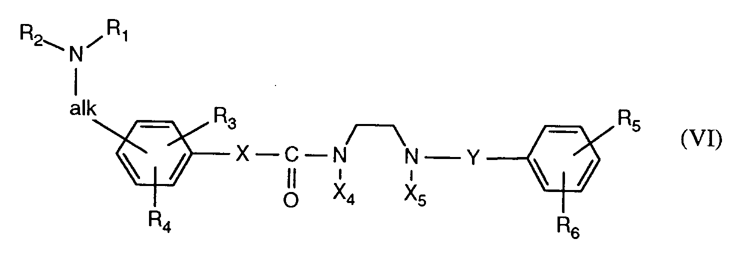 worin einer der Reste X₄ und X₅ Wasserstoff und der andere eine Gruppe der Formel -CH₂-CH₂-X₃ (VIa) bedeutet und X₃ für Hydroxy oder reaktionsfähiges verestertes Hydroxy steht, cyclisiert, oder
d) eine Verbindung der Formel VII, 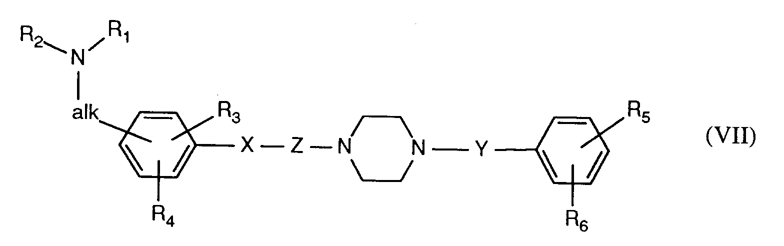 worin Z eine zu Carbonyl oxidierbare Gruppe bedeutet, oxidiert, oder
e) in eine Verbindung der Formel VIII, 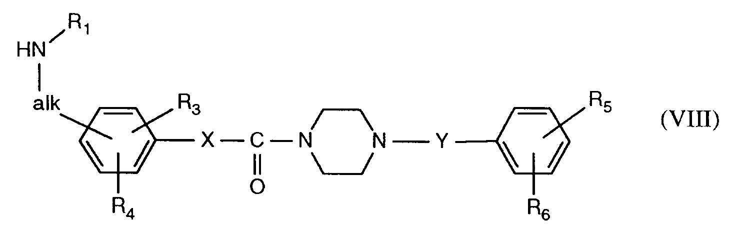 den Rest R₂ einführt (R₂ ≠ H), oder
f) zur Herstellung von Verbindungen, worin R₁ Niederalkoxyniederalkyl bedeutet, in eine Verbindung der Formel IX 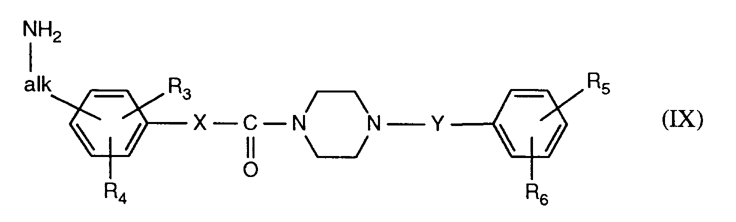 den Rest R₁ einführt, oder
g) ein Amin der Formel X 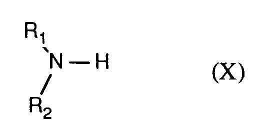 mit einer Verbindung der Formel XI, 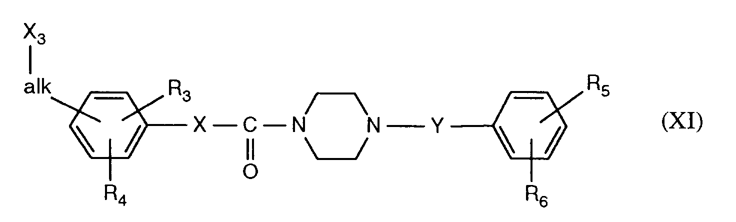 worin X₃ für Hydroxy oder reaktionsfähiges verestertes Hydroxy steht, umsetzt; und jeweils gewünschtenfalls eine verfahrensgemäss oder auf andere Weise erhältliche
Verbindung der Formel I in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensgemäss erhältliches Salz in die freie Verbindung der Formel I oder in ein anderes Salz überführt.

Die vor- und nachstehend beschriebenen Umsetzungen werden in an sich bekannter Weise durchgeführt, z.B. in Ab- oder üblicher Weise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -78° bis zur Siedetemperatur des Reaktionsmediums, vorzugsweise von etwa - 10° bis etwa 150°C, und, falls erforderlich, in einem geschlossenen Gefäss, unter Druck, in einer Inertgasatmosphäre und/oder unter wasserfreien Bedingungen arbeitet.

In den Ausgangsmaterialien kann das basische Zentrum z.B. in Form von Säureadditionssalzen, beispielsweise mit den vorstehend im Zusammenhang mit Salzen von Verbindungen der Formel I aufgeführten Säuren, vorliegen, während Ausgangsverbindungen der Formel II, worin X₁ Carboxy bedeutet, Salze mit Basen bilden können. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Übergangsmetallsalze, wie Zink- oder Kupfersalze, oder Salze mit Ammoniak oder organischen Aminen, wie cyclische Amine, wie Mono-, Di- bzw. Trihydroxy-C₁-C₇-alkylamine, Hydroxy-C₁-C₇-alkyl-C₁-C₇-alkyl-amine oder wie Polyhydroxy-C₄-C₇-alkylamine. Cyclische Amine sind z.B. Morpholin, Thiomorpholin, Piperidin oder Pyrrolidin. Als Mono-C₁-C₇-alkylamine kommen beispielsweise Äthyl- oder tert.- Butylamin, als Di-C₁-C₇-alkylamine beispielsweise Diäthyl- oder Diisopropylamin, als Tri-C₁-C₇-alkylamine beispielsweise Trimethyl- oder Triäthylamin in Betracht. Entsprechende Hydroxy-C₁-C₇-alkylamine sind z.B. Mono-, Di- bzw. Triäthanolamine, und Hydroxy-C₁-C₇-alkyl-C₁-C₇-alkylamine sind z.B. N,N-Dimethylamino- oder N,N-Diäthylaminoäthanol, ferner Glucosamin als Polyhydroxy-C₆-alkylamin.

Reaktionsfähiges funktionell abgewandeltes Carboxy X₁ bedeutet beispielsweise verestertes, in erster Linie reaktionsfähiges verestertes, Carboxy, anhydridisiertes Carboxy oder amidiertes Carboxy.

Verestertes Carboxy ist beispielsweise gegebenenfalls substituiertes C₁-C₇-Alkoxycarbonyl, wie Äthoxycarbonyl, vorzugsweise jedoch reaktionsfähiges verestertes Carboxy, beispielsweise gegebenenfalls, z.B. durch C₁-C₇-Alkoxy oder gegebenenfalls substituiertes Carbamoyl, zusätzlich aktiviertes Vinyloxycarbonyl, wie 1-C₁-C₇-Alkoxy-, z.B. 1-Äthoxyvinyloxycarbonyl, oder 2-(N-C₁-C₇-Alkyl-carbamoyl)-, z.B. 2-(N-Äthylcarbamoyl)-vinyloxycarbonyl, sowie gegebenenfalls, z.B. durch Nitro, Halogen, C₁-C₇-Alkansulfonyl oder Phenylazo, substituiertes Phenoxy- bzw. Thiophenoxycarbonyl, wie 4-Nitro-, 2,4,5-Trichlor-, Pentachlor-, 4-Methansulfonyl-, 4-Phenylazo-phenoxycarbonyl, Thiophenoxy- oder 4-Nitrothiophenoxycarbonyl, und ebenso aktiviertes, z.B. durch Cyano oder ferner gegebenenfalls verestertes Carboxy substituiertes, Methoxycarbonyl, insbesondere Cyanomethoxycarbonyl. Reaktionsfähiges verestertes Carboxy kann ebenso 1,1- oder 1,3-disubstituiertes 2-Isoureidocarbonyl, wie 1,1-Diniederalkyl-, 1,1-Diaryl- oder 1,1-Diaryl-C₁-C₇-alkyl-2-isoureidocarbonyl, z.B. 1,1-Diäthyl-, 1,1-Diphenyl- oder 1,1-Dibenzyl-2-isoureidocarbonyl, oder 1,3-Dicycloalkyl-, z.B. 1,3-Dicyclohexyl-2-isoureido-carbonyl, oder N-C₂-C₇-Alkylenaminooxycarbonyl, wie N-Piperidinyl-oxycarbonyl, sowie N-Imido-oxycarbonyl, z.B. N-Succinimido-oxy- oder N-Phthalimido-oxycarbonyl, sein.

Unter anhydridisiertem Carboxy ist beispielsweise gegebenenfalls verzweigtes C₁-C₇-Alkoxycarbonyloxycarbonyl, wie Äthoxy- oder Isobutyloxycarbonyloxycarbonyl, Halogencarbonyl, wie Chlorcarbonyl, Azidocarbonyl, Halogenphosphoryloxycarbonyl, wie Dichlorphosphoryloxycarbonyl, oder gegebenenfalls, z.B. durch Halogen oder Aryl, substituiertes C₁-C₇-Alkanoyloxycarbonyl, wie Pivaloyloxy-, Trifluoracetyloxy- oder Phenylacetoxycarbonyl, zu verstehen.

Reaktionsfähiges amidiertes Carboxy ist beispielsweise gegebenenfalls, z.B. durch C₁-C₇-Alkyl, substituiertes 1-Imidazolyl- oder 1-Pyrazolylcarbonyl, wie 3,5-Dimethyl-pyrazolylcarbonyl.

Eine Aminoschutzgruppe X₂ ist beispielsweise Acyl, wie C₁-C₇-Alkanoyl, z.B. Formyl oder Acetyl, Halogencarbonyl, wie Chlorcarbonyl, ferner gegebenenfalls substituiertes Aryl- oder Heteroarylsulfonyl, wie 2-Pyridyl- oder 2-Nitrophenylsulfonyl.

Im Rahmen der vor- und nachstehenden Verfahrensbeschreibung bedeutet reaktionsfähiges verestertes Hydroxy, z.B. X₃, sofern nicht abweichend definiert, insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes C₁-C₇-Alkansulfonyloxy, z.B. Methan- oder Trifluormethansulfonyloxy, C₃-C₇-Cycloalkansulfonyloxy, z.B. Cyclohexansulfonyloxy, oder gegebenenfalls, z.B. durch C₁-C₇-Alkyl oder Halogen, substituiertes Benzolsulfonyloxy, z.B. p-Bromphenyl- oder p-Toluolsulfonyloxy.

Werden bei den vor- und nachstehend beschriebenen Umsetzungen beispielsweise Basen verwendet, so kommen, sofern nicht abweichend aufgeführt, beispielsweise Alkalimetallhydroxide, -hydride, -amide, -alkanolate, -carbonate, -triphenylmethylide, -di-C₁-C₇-alkylamide, -amino-C₁-C₇-alkylamide oder -C₁-C₇-alkylsilylamide, Naphthylamine, C₁-C₇-Alkylamine, basische Heterocyclen, Ammoniumhydroxide sowie carbocyclische Amine in Frage. Beispielhaft seien Lithiumhydroxid, Natriumhydroxid, -hydrid, -amid, -äthylat, Kaliumtertiärbutanolat, -carbonat, Lithiumtriphenylmethylid, -diisopropylamid, Kalium-3-(aminopropyl)-amid, -bis-(trimethylsilyl)-amid, Dimethylaminonaphthalin, Di- oder Triäthylamin, Pyridin, Benzyl-trimethylammoniumhydroxid, 1,5-Diaza-bicyclo[4.3.0]non-5-en (DBN) sowie 1,8-Diaza-bicyclo[5.4.0]undec-7-en (DBU) genannt.
Variante a): Die verfahrensgemässe N-Acylierung wird in an sich bekannter Weise durchgeführt, erforderlichenfalls in Gegenwart eines, insbesondere basischen, Kondensationsmittels. Als Basen kommen beispielsweise Vertreter der vorstehend aufgeführten Basen in Frage. Häufig ist auch die Basizität der Verbindung der Formel III ausreichend.
   Falls X₁ Carboxy bedeutet, werden z.B. primär die entsprechenden Ammoniumsalze gebildet, die durch Erwärmen oder Behandeln mit geeigneten Dehydratisierungsmitteln (als Kondensationsmittel), wie Carbodiimiden, z.B. N,N'-Diniederalkyl- oder N,N'-Dicycloalkylcarbodiimid, wie N,N'-Diäthyl-, N,N'-Diisopropyl- oder N,N'-Dicyclohexyl-carhodiimid, vorteilhaft unter Zusatz von N-Hydroxysuccinimid oder gegebenenfalls, z.B. durch Halogen, Niederalkoxy oder Niederalkyl, substituiertem 1-Hydroxy-benzotriazol oder N-Hydroxy-5-norbornen-2,3-dicarboxamid, sowie N,N-Carbonyldiimidazol, dehydratisiert werden können. Mit Carbodiimiden können intermediär z.B. auch die entsprechenden 1-Isoureidocarbonyl-Verbindungen gebildet werden. Als wasserbindende Kondensationsmittel können weiterhin N-Äthoxycarbonyl-2-ethoxy-1,2-dihydrochinolin, Phosphorylcyanamide bzw. -azide, wie Diäthylphosphorylcyanamid oder Diphenylphosphorylazid, Triphenylphosphin-disulfid oder 1-Niederalkyl-2-halogeno-piperidinium-halogenide, wie 1-Methyl-2-chlor- pyridinium-iodid, eingesetzt werden.
   Die in dieser Verfahrensvariante verwendeten Ausgangsstoffe sind z.T. bekannt bzw. können nach an sich bekannten Verfahren hergestellt werden.
   Zur Herstellung von Verbindungen der Formel II, worin X₁ gegebenenfalls substituiertes C₁-C₇-Alkoxycarbonyl bedeutet, kann man üblicherweise von der freien Säure (X₁ = Carboxy) oder einem Säureanhydrid (X₁ bedeutet z.B. Halogencarbonyl) ausgehen und diese beispielsweise mit dem entsprechenden, erforderlichenfalls in reaktionsfähiger Form vorliegenden, Alkohol, z.B. einem C₁-C₇-Alkylhalogenid, umsetzen. Die Herstellung von Verbindungen der Formel II, worin X₁ gegebenenfalls zusätzlich aktiviertes Vinyloxycarbonyl bedeutet, kann z.B. durch Umesterung eines C₁-C₇-Alkylesters mit Vinylacetat (Methode des aktivierten Vinylesters), durch Umsetzung der freien Säure von Verbindungen der Formel II mit Niederalkoxyacetylen (z.B. Äthoxyacetylen-Methode) oder, analog der Woodward-Methode, mit einem 1,2-Oxazoliumsalz erfolgen. Gegebenenfalls substituiertes Phenoxy- bzw. Thiophenoxycarbonyl aufweisende Verbindungen der Formel II können beispielsweise ausgehend von der freien Säure nach der Carbodiimid-Methode durch Umsetzen mit dem entsprechenden (Thio-)Phenol durchgeführt werden. Ebenfalls ausgehend von der freien Säure der Formel II können Verbindungen der Formel II, worin X₁ aktiviertes Methoxycarbonyl bzw. 1,1- oder 1,3-disubstituiertes 2-Isoureidocarbonyl darstellt, z.B. durch Umsetzung mit einem Halogen-, wie Chloracetonitril (Cyanmethylester-Methode) bzw. mit einem Carbodiimid oder Cyanamid (Carbodiimid- oder Cyanamid-Methode) erhalten werden.
   Die Herstellung von N-C₂-C₇-Alkylenamino-oxycarbonyl- bzw. N-Imido-oxycarbonyl-Verbindungen der Formel II kann beispielsweise bei Verwendung der freien Säure der Formel II aus entsprechenden N-Hydroxyverbindungen mit Hilfe von Carbodiimiden nach der Methode der aktivierten N-Hydroxyester erfolgen. Zur Herstellung von Verbindungen der Formel II, worin X₁ gegebenenfalls verzweigtes C₁-C₇-Alkoxycarbonyloxycarbonyl, Halogenphosphoryloxycarbonyl bzw. gegebenenfalls substituiertes C₁-C₇-Alkanoyloxycarbonyl bedeutet, kann beispielsweise von freier Säure der Formel II ausgegangen werden, die z.B. mit einen, entsprechenden Halogenid, wie gegebenenfalls substituiertes C₁-C₇-Alkylkohlensäurehalogenid (Methode der gemischten (O-Kohlensäureanhydride), Phosphoroxyhalogenid (z.B. Phosphoroxychlorid-Methode) oder gegebenenfalls substituiertes C₁-C₇-Alkanoylhalogenid (Methode der gemischten Carbonsäurehalogenide) behandelt werden kann. Azidocarbonylverbindungen der Formel II sind beispielsweise durch Behandeln entsprechender Hydrazide mit salpetriger Säure zugänglich (Azid-Methode). Zur Herstellung von Verbindungen der Formel II, worin X₁ gegebenenfalls substituiertes 1-Imidazolyl-carbonyl bzw. 1-Pyrazolyl-carbonyl bedeutet, setzt man die freie Säure der Formel II z.B. mit Di-(1-imidazolyl)-carbonyl (Imidazolid-Methode) bzw. das betreffende Hydrazid z.B. mit einem entsprechenden 1,3-Diketon (Pyrazolid-Methode) um.
Variante b): Der Rest X₃ bedeutet insbesondere reaktionsfähiges verestertes Hydroxy, z.B. Halogen, wie Chlor.
   Die verfahrensgemässe N-Alkylierung wird in an sich bekannter Weise durchgeführt, erforderlichenfalls in Gegenwart einer, z.B. der vorstehend aufgeführten, Basen.
   Die in dieser Verfahrensvariante verwendeten Ausgangsstoffe sind z.T. bekannt oder können in an sich bekannter Weise hergestellt werden.
   So kann beispielsweise das Ausgangsmaterial der Formel IV hergestellt werden, indem man eine Verbindung der Formel II oder ein Salz davon, worin X₁ Carboxy oder reaktionsfähiges funktionell abgewandeltes Carboxy bedeutet, mit einer Verbindung der Formel IVa 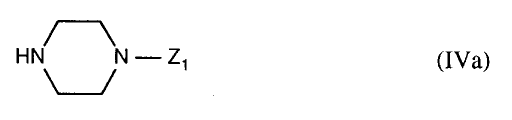 oder einem Salz davon, worin Z₁ Wasserstoff oder eine Aminoschutzgruppe, wie Benzyl, bedeutet, in der in Variante a) beschriebenen Weise umsetzt und gegebenenfalls die Aminoschutzgruppe, z.B. Benzyl durch übliche Hydrogenolyse, abspaltet.
   Variante c): Die verfahrensgemässe Cyclisierung (intramolekulare N-Alkylierung) wird in an sich bekannter Weise, erforderlichenfalls in Gegenwart eines, insbesondere basischen, Kondensationsmittels durchgeführt. Als Basen werden z.B. vorstehend aufgeführten verwendet.
   X₃ bedeutet dabei insbesondere reaktionsfähiges verestertes Hydroxy, bevorzugt Halogen, wie Chlor.
   Das Ausgangsmaterial kann in an sich bekannter Weise hergestellt werden. Beispielsweise geht man von einer Verbindung der Formel II oder einem Salz davon aus, worin X₁ Carboxy oder reaktionsfähiges funktionell abgewandeltes Carboxy bedeutet, und setzt diese zunächst mit einer Verbindung der Formel 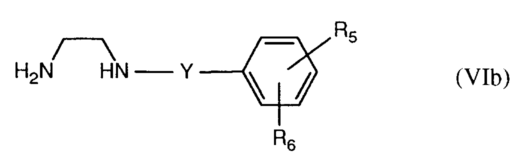 in Analogie zu Variante a) um. Im nächsten Reaktionsschritt wird die erhaltene Verbindung mit einer Verbindung der Formel X₃-CH₂-CH₂-X₃ (VIc) unter N-alkylierenden Bedingungen gemäss Variante b) umgesetzt.
Variante d): Eine zu -CO- oxidierbare Gruppe Z steht in erster Linie für -CH₂₋. Die Oxidation entsprechender Verbindungen der Formel VII erfolgt mit Hilfe eines geeigneten Oxidationsmittels, wobei bevorzugt gegebenenfalls, z.B. durch einen Phenylrest, substituierte Tetra-C₁-C₄-alkylammoniumpermanganate, in erster Linie Benzyl-triäthylammoniumpermanganat, verwendet wird.
   Das Ausgangsmaterial der Formel VII wird in an sich bekannter Weise hergestellt. Beispielsweise geht man von einer Verbindung der Formel III aus, worin X₂ Wasserstoff bedeutet, und setzt diese unter den in Variante b) beschriebenen N-alkylierenden Bedingungen mit einer Verbindung der Formel VIIa 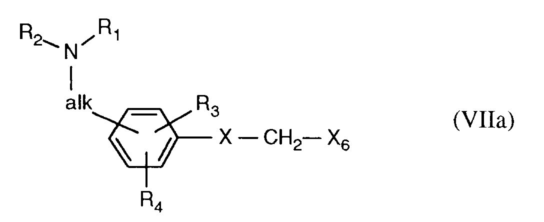 um, wobei X₆ Hydroxy oder in erster Linie reaktionsfähiges verestertes Hydroxy, insbesondere Halogen, wie Chlor oder Brom, bedeutet.
Variante e): Die Einführung von Niederalkenoyl bzw. elektronegativ substituiertem Niederalkanoyl (N-Acylierung) erfolgt in üblicher Weise, erforderlichenfalls in Gegenwart eines, insbesondere basischen, Kondensationsmittels. Als Basen kommen beispielsweise Vertreter der vorstehend aufgeführten Basen in Frage.
Variante f): Die Einführung von Niederalkoxyniederalkyl R₁ (N-Alkoxyalkylierung) erfolgt in üblicher Weise, erforderlichenfalls in Gegenwart eines, insbesondere basischen, Kondensationsmittels. Als Basen kommen beispielsweise Vertreter der vorstehend aufgeführten Basen in Frage.
Variante g): Bei der Variante g) handelt es sich um die an sich bekannte N-Alkylierung von Ammoniak bzw. primären oder sekundären Aminen. Mit umfasst sind auch alle Varianten, die eine gezielte Synthese von primären, sekundären und tertiären Aminen auf diesem Wege ermöglichen. Reaktionsfähiges verestertes Hydroxy X₃ bedeutet dabei z.B. Halogen, wie Chlor.

Eine verfahrensgemäss erhältliche erfindungsgemässe Verbindung kann in an sich bekannter Weise in eine andere erfindungsgemässe Verbindung übergeführt werden.

In erfindungsgemässen Verbindungen, in welchen R₁ Niederalkoxyniederalkyl und R₂ Wasserstoff ist, kann die Aminogruppe in der vorstehend unter Variante a), b) oder e) angegebenen Weise N-acyliert werden. Ebenso kann eine Verbindung der Formel I, worin R₁ Wasserstoff und R₂ Niederalkenoyl oder elektronegativ substituiertes Niederalkanoyl bedeutet, entsprechend der für die Verfahrensvariante f) angegebenen Weise N-niederalkyliert oder durch Niederalkoxyniederalkyl N-substituiert werden. Die N-Niederalkylierung kann dabei auch analog der Leuckart-Wallach- (bzw. Eschweiler-Clarke)-Reaktion aus Carbonylverbindungen, z.B. unter Verwendung von Ameisensäure als Reduktionsmittel, reduktiv durchgeführt werden.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyäthylenglykol, aufweisen. Tabletten können ebenfalls Bindemittel, z.B. Magnesiumaluminiunisilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I z.B. in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgenden Beispiele dienen zur Illustration der Erfindung; Temperaturen sind in Celsiusgraden, Drucke in mbar angegeben. Folgende Abkürzungen werden verwendet: RT = Raumtemperatur, Aether = Diäthyläther, DMF = Dimethylformamid, Essigester = Essigsäureäthylester, (BOC)₂O = Di-tert.-Butyl-dicarbonat.

### Beispiel 1: 1-{4-[N-(2-Isopropyloxyäthyl)-N-chloracetyl-aminomethyl]-benzoyl}-4-[2-(4-chlorphenyl)-äthyl]-piperazin-hydrochlorid

1,43 g 1-[2-(4-Chlorphenyl)-äthyl]-piperazin werden mit 0,83 g N-Aethyldiisopropylamin (Hünig-Base) in 35 ml Methylenchlorid gelöst. Dazu werden 2 g rohes 4-[N-(2-Isopropyloxyäthyl)-N-chloracetyl-aminomethyl]-benzoesäurechlorid in etwa 10 ml Methylenchlorid getropft. Die so erhaltene klare Lösung wird über Nacht bei RT gerührt. Dann wird sie mit Wasser gewaschen, über Natriumsulfat getrocknet und zur Trockene eingedampft. Das rohe Öl (3,2 g) wird an Kieselgel chromatographiert (Elutionsmittel: Methylenchlorid/Aceton 500:150 und Methylenchlorid/Aceton/Methanol 500:100:25) und als Hydrochlorid aus Aethanol/Aether kristallisiert. Man erhält die Titelverbindung vom Smp. 186-188°.

Das Ausgangsmaterial wird wie folgt hergestellt:
(a) 30 g 4-Aminomethyl-benzoesäure (Fa. Janssen) werden in je 250 ml Acetanhydrid und Pyridin suspendiert und bei 60° Aussentemperatur gerührt, wobei nach etwa 45 min die 4-Aminomethylbenzoesäure in Lösung geht. Nach 2h wird die Heizung entfernt, und man rührt bei RT über Nacht. Die Lösung wird eingedampft. Das kristalline Material wird mit 400 ml Wasser versetzt; die erhaltene Suspension wird mit 2N Salzsäure angesäuert, 30 Min. kräftig gerührt und abgenutscht. Die mit Wasser nachgewaschenen farblosen Kristalle werden in 250 ml Aethanol gelöst. Diese Lösung wird über Natriumsulfat getrocknet und mit 200 ml Aether verdünnt. Nach Abkühlen kristallisiert die 4-Acetylaminomethyl-benzoesäure vom Smp. 196-200°.
(b) 2,5 g 4-Acetylaminomethyl-benzoesäure werden in 20 ml abs. Aethanol warm gelöst. Dazu gibt man 0.3 ml konz. Schwefelsäure und kocht 10h am Rückfluss. Die Lösung wird zur Trockene eingedampft. Der Rückstand wird in Essigester gelöst und mit Sodalösung und Wasser gewaschen, getrocknet und eingedampft. Der aus Essigester/Petroläther umkristallisierte 4-Acetylaminomethyl-benzoesäureäthylester schmilzt bei 100-101°.
(c) 6,6 g des gemäss (b) erhaltenen Esters werden in 60 ml Acetonitril gelöst und mit 0,36 g 4-Dimethylaminopyridin versetzt. Dazu tropft man innerhalb von 5 min 8 g (BOC)₂O in 10 ml Acetonitril. Man lässt über Nacht bei RT stehen. Dabei erhält man den 4-(N-Acetyl-N-t-butyloxycarbonyl-aminomethyl)-benzoesäureäthylester, der in Lösung vorliegt. Man gibt zu dieser Lösung 5,9 g 2-Diäthylaminoäthylamin und lässt das Gemisch 5h stehen. Die weiteren Arbeitsschritte sind: eindampfen, den Rückstand in Essigester aufnehmen, mit Kochsalz-Lösung und Wasser waschen, wieder eindampfen, an Kieselgel chromatographieren (Elutionsmittel: Methylenchlorid und Methylenchlorid/Aceton 4:1) und aus Aether/Petroläther umkristallisieren. So erhält man den 4-t-Butyloxycarbonyl-aminomethyl-benzoesäureäthylester, Smp. 96-97°.
(d) 5,9 g 4-t-Butyloxycarbonyl-aminomethyl-benzoesäureäthylester werden in 50 ml DMF gelöst. Man gibt 1,5 g pulv. KOH dazu. Man rührt 15 min bei 55° und gibt man 5,5 g O-(2-Isopropyloxyäthyl)-tosylat hinzu. Man rührt 5h bei 60-65°. Die Lösung wird eingedampft. Man erhält so das ölige 4-[N-(2-Isopropyloxyäthyl)-N-t-butyloxycarbonyl-aninomethyl]-benzoesäureäthylester. Es wird ohne Reinigung weiterverwendet.
(e) 7,1 g des rohen 4-[N-(2-Isopropyloxyäthyl)-N-t-butyloxycarbonyl-aminomethyl]-benzoesäureäthylesters werden in 70 ml Methylenchlorid gelöst und mit 40 ml Trifluoressigsäure versetzt. Die klare Lösung wird über Nacht bei RT stehen gelassen und dann eingedampft. Der Rückstand wird in Methylenchlorid aufgenommen, mit Wasser-Ammoniak (pH > 9) gewaschen und eingedampft. Der ölig-kristalline Rückstand wird in 50 ml Aethanol gelöst und mit 50 ml 2N Natronlauge versetzt. Nach 2h Rühren bei 60-65° wird die Lösung eingedampft und der Rückstand mit Wasser und 2N Salzsäure auf pH 1 gestellt. Es ensteht sofort eine starke Trübung und nach etwa 5 min ein Niederschlag. Man dampft ein und erwärmt den Rückstand kurz mit 100 ml Aethanol. Die Lösung wird mit Magnesiumsulfat getrocknet, über Hyflo filtriert und eingedampft. Das rohe kristalline Material wird in 80 ml Tetrahydrofuran suspendiert und mit 3,2 g Chloracetylchlorid versetzt. Diese Suspension wird 14h bei RT gerührt, dann zur Trockene eingedampft. Der Rückstand wird in Methylenchlorid gelöst, mit verdünnter Salzsäure gewaschen, getrocknet und eingedampft. Man erhält klebrige, schwer lösliche Kristalle, die der 4-[N-(2-Isopropyloxyäthyl)-N-chloracetyl-aminomethyl]-benzoesäure entsprechen.
(f) 2 g der unter (e) erhaltenen Säure werden in 35 ml Methylenchlorid suspendiert, mit 1,1 g Thionylchlorid und 2 Tropfen Pyridin versetzt und über Nacht gerührt. Die leicht trübe Lösung wird über Hyflo filtriert und eingedampft. Man erhält so das ölige 4-[N-(2-Isopropyloxyäthyl)-N-chloracetyl-aminomethyl]-benzoesäurechlorid.

### Beispiel 2: 1-{4-[2-(N-Chloracetyl-aminoäthyl]-benzoyl}-4-[2-(4-chlorphenyl)-äthyl]-piperazin-hydrochlorid

0,38 g 1-[2-(4-Chlorphenyl)-äthyl]-piperazin werden mit 0,22 g Hünig-Base in 10 ml Methylenchlorid gelöst. Dazu wird eine Lösung von 0,44 g rohem 4-[2-(N-Chloracetyl-aminoäthyl)]-benzoesäurechlorid in etwa 10 ml Methylenchlorid getropft. Die so erhaltene klare Lösung wird über Nacht bei RT gerührt. Die Methylenchlorid-Lösung wird mit Wasser gewaschen, über Natriumsulfat getrocknet und zur Trockene eingedampft. Das rohe Öl (3,2 g) wird an Kieselgel chromatographiert (Elutionsmittel: Methylenchlorid/Methanol 250:10) und als Hydrochlorid aus Aethanol und Isopropanol kristallisiert. Man erhält so die Titelverbindung vom Smp. 215-216°.

Das Ausgangsmaterial wird wie folgt hergestellt:
(a) 0,4 g 4-[2-(N-Chloracetyl-aminoäthyl)]-benzoesäure [Fujii et al., J. Pharm. Sci. 66 (1977) 844-848] werden in 10 ml Tetrahydrofuran gelöst. Dazu gibt man 0,3 g Thionylchlorid und 2 Tropfen Pyridin. Die Suspension wird 4h bei RT gerührt, eingedampft und gut getrocknet. Das so erhaltene rohe 4-[(2-N-Chloracetyl-aminoäthyl)]-benzoesäurechlorid wird ohne Reinigung weiter verwendet.

### Beispiel 3:

Tabletten, enthaltend je 50 mg 1-{4-[N-(2-Isopropyloxyäthyl)-N-chloracetyl-aminomethyl]-benzoyl}-4-[2-(4-chlorphenyl)-äthyl]-piperazin, oder ein Salz davon, z.B. das Hydrochlorid, werden wie folgt hergestellt:

| Zusammensetzung (10000 Tabletten) | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Äthanol | q.s. |

Der Wirkstoff wird mit der Lactose und 292 g Kartoffelstärke vermischt, die Mischung mit einer äthanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

### Beispiel 4:

Gelatinesteckkapseln, enthaltend 100 mg Wirkstoff, z.B. 1-{4-[N-(2-Isopropyloxyäthyl)-N-chloracetyl-aminomethyl]-benzoyl}-4-[2-(4-chlorphenyl)-äthyl]-piperazin, oder ein Salz davon, z.B. das Hydrochlorid, werden wie folgt hergestellt:

| Zusammensetzung (für 1000 Kapseln) | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g. |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

### Beispiel 5:

Lacktabletten, enthaltend je 100 mg 1-{4-[N-(2-Isopropyloxyäthyl)-N-chloracetyl-aminomethyl]-benzoyl}-4-[2-(4-chlorphenyl)-äthyl]-piperazin, oder ein Salz davon, z.B. das Hydrochlorid, werden wie folgt hergestellt:

| Zusammensetzung (für 1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Methylenchlorid | q.s. . |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das

Calciumstearat zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

### Beispiel 6:

Eine 0,2%-ige Injektions- oder Infusionslösung von 1-{4-[N-(2-Isopropyloxyäthyl)-N-chloracetyl-aminomethyl]-benzoyl)-4-[2-(4-chlorphenyl)-äthyl]-piperazin, oder einem Salz davon, z.B. dem Hydrochlorid, wird wie folgt hergestellt:

| Zusammensetzung (für 1000 Ampullen) | |
|---|---|
| Wirkstoff | 5,0 g |
| Natriumchlorid | 22,5 g |
| Phosphatpuffer pH= 7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

### Beispiel 7:

In analoger Weise wie in Beispiel 3 bis 6 beschrieben, können auch pharmazeutische Präparate, die jeweils eine andere der in den Beispielen 1 bis 2 genannten Verbindungen enthalten, hergestellt werden.

## Patentansprüche

1. Verbindungen der Formel I, 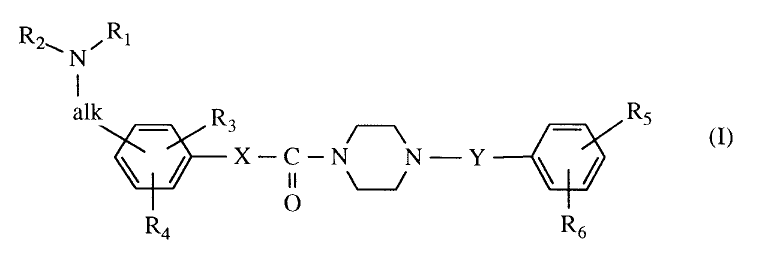 worin alk Niederalkylen bedeutet, R₁ für Wasserstoff, Niederalkyl, Niederalkoxy-niederalkyl, Niederalkoxy-niederalkoxy-niederalkyl, Aryloxy-niederalkyl, Arylnieder-alkoxy-niederalkyl, N-Niederalkylamino-niederalkyl oder N,N-Diniederalkylamino-niederalkyl steht, R₂ Niederalkenoyl, (Carboxy oder funktionell modifiziertes Carboxy)-niederalkenoyl, elektronegativ substituiertes Niederalkanoyl, Carbamoyl, N-Niederalkylcarbamoyl, N,N-Diniederalkylcarbamoyl, Carboxy-carbonyl, Niederalkoxycarbonyl-carbonyl, (Carbamoyl, N-Niederalkylcarbamoyl, N,N-Diniederalkylcarbamoyl)-carbonyl, Wasserstoff, Niederalkyl, Niederalkanoyl, Carboxy, Niederalkoxycarbonyl oder Arylniederalkoxycarbonyl bedeutet, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, und X und Y unabhängig voneinander eine direkte Bindung, Niederalkylen oder Niederalkenylen bedeuten, und Salze davon.

2. Verbindungen der Formel I gemäss Anspruch 1, worin alk Niederalkylen bedeutet, R₁ für Wasserstoff, Niederalkyl, Niederalkoxy-niederalkyl, Niederalkoxy-niederalkoxy-niederalkyl; Phenyloxy-niederalkyl oder Phenylniederalkoxy-niederalkyl, wobei in den beiden letztgenannten Resten die Phenylgruppe jeweils unsubstituiert oder durch Niederalkyl, Trifluormethyl, Niederalkoxy, Halogen und/oder Hydroxy substituiert ist; N-Niederalkylamino-niederalkyl oder N,N-Diniederalkylamino-niederalkyl steht, R₂ Niederalkenoyl, (Carboxy, Niederalkoxycarbonyl, Carbamoyl, N-Niederalkylcarbamoyl, N,N-Diniederalkylcarbamoyl oder Cyano)-niederalkenoyl, Halogen-niederalkanoyl, Amino-niederalkanoyl, N-Niederalkylamino-niederalkanoyl, N-[(Carboxy oder Niederalkoxycarbonyl)-niederalkylamino]-niederalkanoyl, N,N-Diniederalkylamino-niederalkanoyl, C₄-C₆-Niederalkylenamino-niederalkanoyl, Morpholino-niederalkanoyl, Thiomorpholino-niederalkanoyl; Piperazino-niederalkanoyl, wobei der Piperazinorest unsubstituiert oder in 4-Stellung durch Niederalkyl oder Niederalkanoyl substituiert ist; Hydroxy-niederalkanoyl, Niederalkoxy-niederalkanoyl, Niederalkanoyloxy-niederalkanoyl, Niederalkylthio-niederalkanoyl, Niederalkylsulfinyl-niederalkanoyl, Niederalkylsulfonyl-niederalkanoyl; Phenylthio-niederalkanoyl, Phenylsulfinyl-niederalkanoyl oder Phenylsulfonyl-niederalkanoyl, wobei in den drei letztgenannten Resten die Phenylgruppe jeweils unsubstituiert oder durch Niederalkyl, Niederalkoxy, Halogen und/oder Hydroxy substituiert ist; Carboxy-niederalkanoyl, Niederalkoxy-carbonyl-niederalkanoyl, Carbamoyl-niederalkanoyl, N-Niederalkylcarbamoyl-niederalkanoyl, N,N-Diniederalkylcarbamoyl-niederalkanoyl, Cyano-niederalkanoyl, Carbamoyl, N-Niederalkylcarbamoyl, N,N-Diniederalkylcarbamoyl, Carboxy-carbonyl, Niederalkoxycarbonyl-carbonyl, (Carbamoyl, N-Niederalkylcarbamoyl, N,N-Diniederalkylcarbamoyl)-carbonyl, Wasserstoff, Niederalkanoyl, Carboxy, Niederalkoxycarbonyl oder Phenylniederalkoxycarbonyl, worin die Phenylgruppe unsubstituiert oder durch Niederalkyl, Niederalkoxy, Halogen und/oder Hydroxy substituiert ist, bedeutet, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, und X und Y unabhängig voneinander eine direkte Bindung, Niederalkylen oder Niederalkenylen bedeuten, und Salze davon.

3. Verbindungen der Formel I gemäss Anspruch 1, worin alk Niederalkylen bedeutet, R₁ für Wasserstoff, Niederalkyl, Niederalkoxy-niederalkyl, Niederalkoxy-niederalkoxy-niederalkyl, Phenyloxy-niederalkyl, N-Niederalkylamino-niederalkyl oder N,N-Diniederalkylamino-niederalkyl steht, R₂ Niederalkenoyl, (Carboxy oder Niederalkoxycarbonyl)-niederalkenoyl, Halogen-niederalkanoyl, N-Niederalkylamino-niederalkanoyl, N-[(Carboxy oder Niederalkoxycarbonyl)-niederalkylamino]-niederalkanoyl, N,N-Diniederalkylamino-niederalkanoyl, Piperidino-niederalkanoyl, Hydroxy-niederalkanoyl, Niederalkoxy-niederalkanoyl, Niederalkanoyloxy-niederalkanoyl, Niederalkylthio-niederalkanoyl, Niederalkylsulfinyl-niederalkanoyl, Niederalkylsulfonyl-niederalkanoyl, Phenylthio-niederalkanoyl, Phenylsulfinyl-niederalkanoyl, Phenylsulfonyl-niederalkanoyl, Carboxy-niederalkanoyl, Niederalkoxycarbonyl-niederalkanoyl, Wasserstoff oder Niederalkanoyl bedeutet, R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Trifluormethyl, Niederalkoxy, Niederalkylthio, Halogen oder Diniederalkylamino stehen, und X und Y unabhängig voneinander eine direkte Bindung, Niederalkylen oder Niederalkenylen bedeuten, und Salze davon.

4. Verbindungen der Formel I gemäss Anspruch 1, worin alk Methylen oder 1,2-Aethylen bedeutet, R₁ für Wasserstoff, Niederalkyl oder Niederalkoxy-niederalkyl steht, R₂ Niederalkenoyl, (Carboxy oder Niederalkoxycarbonyl)-niederalkenoyl oder Halogen-niederalkanoyl bedeutet oder R₂, sofern R₁ für Niederalkoxy-niederalkyl steht, auch Wasserstoff bedeuten kann, R₃ und R₄ unabhängig voneinander Wasserstoff, Niederalkyl, Halogen, Niederalkoxy oder Niederalkylthio bedeuten, R₅ für Niederalkylthio, Chlor, Fluor oder Brom steht, R₆ Wasserstoff bedeutet, X für eine direkte Bindung oder 1,2-Aethenylen steht und Y 1,2-Aethylen bedeutet, und pharmazeutisch verwendbare Salze davon.

5. Verbindungen gemäss Anspruch 1 von der Formel Ia, 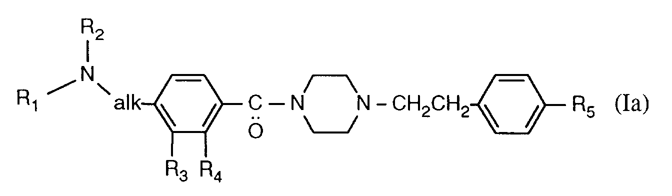 worin alk Niederalkylen bedeutet, R₁ für Wasserstoff, Niederalkyl oder Niederalkoxy-niederalkyl steht, R₂ Niederalkenoyl, (Carboxy oder Niederalkoxycarbonyl)-niederalkenoyl oder durch Halogen, Diniederalkylamino, 4- bis 6-gliedriges Niederalkylenamino oder Niederalkylthio substituiertes Niederalkanoyl bedeutet oder R₂, sofern R₁ Niederalkoxyniederalkyl darstellt, Wasserstoff bedeuten kann, R₃ und R₄ unabhängig voneinander Wasserstoff, Niederalkyl, Chlor, Fluor oder Brom bedeuten und R₅ Niederalkylthio, Chlor, Fluor oder Brom darstellt, und Salze davon.

6. Verbindungen der Formel Ia gemäss Anspruch 5, worin alk für C₁-C₄-Alkylen steht, R₁ Wasserstoff, C₁-C₄-Alkyl oder C₁-C₄-Alkoxy-C₂-C₄-alkyl bedeutet, R₂ C₃-C₇-Alkenoyl, (Carboxy oder C₁-C₄-Alkoxycarbonyl)-C₃-C₇-alkenoyl, Chlor-C₂-C₄-alkanoyl, C₁-C₄-Alkylthio-, C₁-C₄-Alkylsulfinyl- oder C₁-C₄-Alkylsulfonyl-C₂-C₄-alkanoyl bedeutet oder R₂, sofern R₁ für C₁-C₄-Alkoxy-C₂-C₄alkyl steht, auch Wasserstoff darstellen kann, R₃ und R₄ unabhängig voneinander Wasserstoff, C₁-C₄-Alkyl, Fluor oder Chlor bedeuten und R₅ für Chlor oder Brom steht, und pharmazeutisch verwendbare Salze davon.

7. Verbindungen der Formel Ia gemäss Anspruch 5, worin alk für Methylen oder 1,2-Aethylen steht, R₁ Wasserstoff, Methyl, Äthyl oder 2-Isopropyloxyäthyl bedeutet, R₂ Prop-2-enoyl, 3-(Carboxy oder C₁-C₄-Alkoxycarbonyl)-prop-2-enoyl, Chloracetyl, 3-Chlorpropionyl, Methylthioacetyl oder Äthylthioacetyl darstellt, R₃ und R₄ unabhängig voneinander Wasserstoff, Methyl, Fluor oder Chlor bedeuten und R₅ für Chlor oder Brom steht, und pharmazeutisch verwendbare Salze davon.

8. Verbindungen der Formel Ia gemäss Anspruch 5, worin alk für Methylen oder 1,2-Aethylen steht, R₁ Wasserstoff oder 2-Isopropyloxyäthyl bedeutet, R₂ Chloracetyl darstellt, R₃ und R₄ unabhängig voneinander Wasserstoff, Methyl, Fluor oder Chlor bedeuten und R₅ für Chlor oder Brom steht, und pharmazeutisch verwendbare Salze davon.

9. 1-{4-[N-(2-Isopropyloxyäthyl)-N-chloracetyl-aminomethyl]-benzoyl}-4-[2-(4-chlorphenyl)-äthyl]-piperazin gemäss Anspruch 1, oder ein pharmazeutisch anwendbares Salz davon.

10. 1-{4-[2-(N-Chloracetyl-aminoäthyl)]-benzoyl}-4-[2-(4-chlorphenyl)-äthyl]-piperazin gemäss Anspruch 1, oder ein pharmazeutisch anwendbares Salz davon.

11. Pharmazeutische Präparate enthaltend eine Verbindung gemäss einem der Ansprüche 1 bis 10 und mindestens ein pharmazeutisch verwendbares Trägermaterial.

12. Eine Verbindung gemäss einem der Ansprüche 1 bis 10 zur Anwendung in einem Verfahren zur therapeutischen Behandlung des tierischen oder menschlichen Körpers.

13. Eine Verbindung gemäss einem der Ansprüche 1 bis 10 zur Verwendung bei der Behandlung von Krankheiten, die auf eine Hemmung von Interleukin-1 ansprechen.

14. Verwendung einer Verbindung gemäss einem der Ansprüche 1 bis 10 zur Herstellung pharmazeutischer Präparate.

15. Verwendung einer Verbindung gemäss einem der Ansprüche 1 bis 10 zur Herstellung pharmazeutischer Präparate für die Behandlung von Krankheiten, die auf eine Hemmung von Interleukin-1 ansprechen.

16. Verfahren zur Herstellung einer Verbindung der Formel I gemäss Anspruch 1, dadurch gekennzeichnet, dass man
a) eine Verbindung der Formel II, 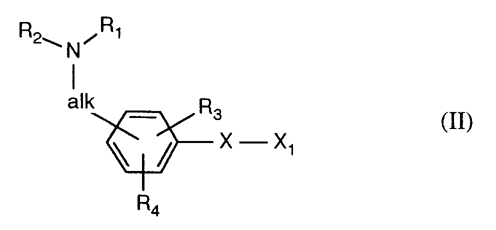 worin X₁ Carboxy oder reaktionsfähiges funktionell abgewandeltes Carboxy bedeutet, oder ein Salz davon, mit einer Verbindung der Formel III, 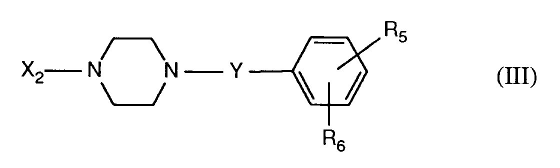 worin X₂ Wasserstoff oder eine Aminoschutzgruppe bedeutet, umsetzt, oder
b) eine Verbindung der Formel IV, 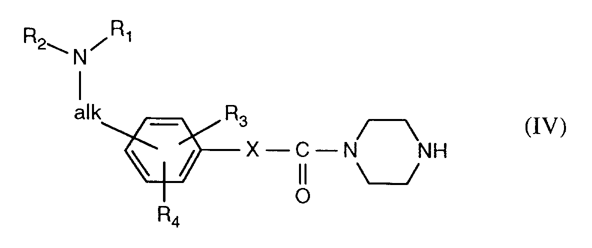 oder ein Salz davon, mit einer Verbindung der Formel V, 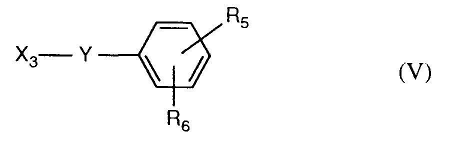 worin X₃ Hydroxy oder reaktionsfähiges verestertes Hydroxy bedeutet, umsetzt, oder
c) eine Verbindung der Formel VI, 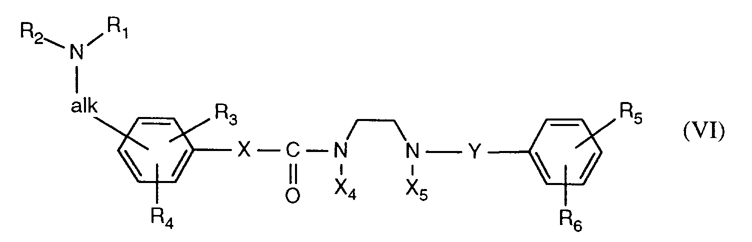 worin einer der Reste X₄ und X₅ Wasserstoff und der andere eine Gruppe der Formel -CH₂-CH₂-X₃ (VIa) bedeutet und X₃ für Hydroxy oder reaktionsfähiges verestertes Hydroxy steht, cyclisiert, oder
d) eine Verbindung der Formel VII, 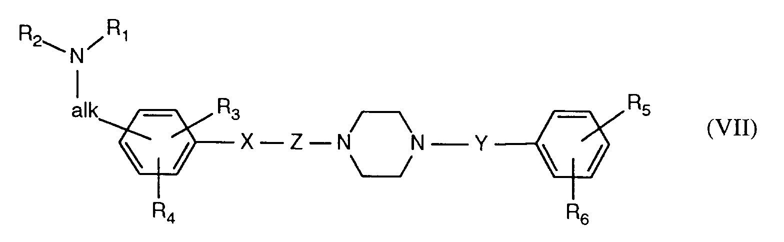 worin Z eine zu Carbonyl oxidierbare Gruppe bedeutet, oxidiert, oder
e) in eine Verbindung der Formel VIII, 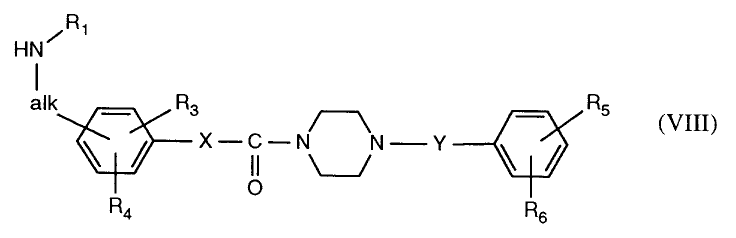 den Rest R₂ einführt (R₂ ≠ H), oder
f) zur Herstellung von Verbindungen, worin R₁ Niederalkoxyniederalkyl bedeutet, in eine Verbindung der Formel IX 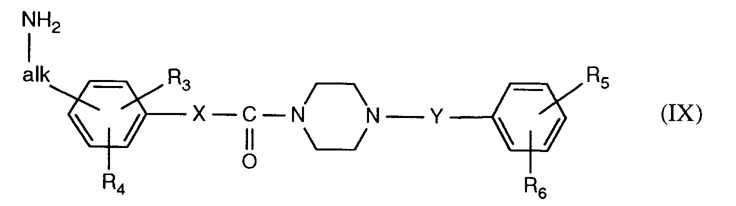 den Rest R₁ einführt, oder
g) ein Amin der Formel X 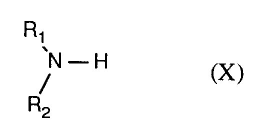 mit einer Verbindung der Formel XI, 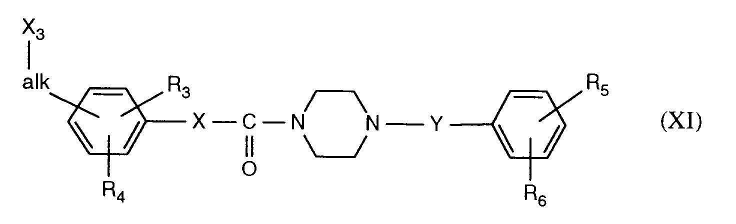 worin X₃ für Hydroxy oder reaktionsfähiges verestertes Hydroxy steht, umsetzt; und jeweils gewünschtenfalls eine verfahrensgemäss oder auf andere Weise erhältliche Verbindung der Formel I in eine andere Verbindung der Formel I überführt, ein verfahrensgemäss erhältliches Isomerengemisch in die Komponenten auftrennt, eine verfahrensgemäss erhältliche freie Verbindung der Formel Ein ein Salz überführt und/oder ein verfahrensgemäss erhältliches Salz in die freie Verbindung der Formel I oder in ein anderes Salz überführt.
